# EUROPEAN PATENT SPECIFICATION

(11) **EP 2 344 226 B1**
(45) Date of publication and mention of the grant of the patent: **28.12.2016**
(21) Application number: 09790952.7
(22) Date of filing: 29.07.2009
(51) Int. Cl.: A61M 25/00

(54) **CATHETER SHAFT BOND ARRANGEMENTS AND METHODS**
VERBINDUNGSANORDNUNG UND VERFAHREN FÜR KATHETERSCHAFT
AGENCEMENTS DE LIAISON DE TIGES DE CATHÉTER ET PROCÉDÉS

(30) Priority: 01.08.2008 US 184268
(43) Date of publication of application: 20.07.2011
(73) Proprietor: Boston Scientific Scimed, Inc., Maple Grove, MN 55311 (US)
(72) Inventor: GUNDERSON, Richard, C., Maple Grove MN 55311 (US); LUCAS, Robert, Princeton MN 55371 (US); TEGELS, Zachary, J., Minneapolis MN 55408 (US); SAGEDAHL, Mark, Alexandria, MN 55308 (US); PRINDLE, Katherine, Robbinsdale MN 55422 (US)
(74) Representative: Vossius & Partner Patentanwälte Rechtsanwälte mbB
(86) International application number: PCT/US2009/052144
(87) International publication number: WO 2010/014741

(56) References cited:
- EP-A- 1 060 757
- EP-A- 1 754 918
- WO-A-01/56731
- WO-A-95/11400
- KR-A- 20040 025 361
- US-A- 3 432 129

## Description

### TECHNICAL FIELD

This disclosure relates to catheter systems and related methods of operating and manufacturing catheter systems. Preferred arrangements also relate to catheter shaft bond arrangement and related methods.

The present invention is set out in the appended claims.

### BACKGROUND

Catheters are used with stents and balloon inflatable structures to treat conditions such as strictures, stenoses, and narrowing in various parts of the body. Various catheter designs have been developed for the dilatation of stenoses and to deliver and deploy stents at treatment sites within the body.

Stents are typically intraluminally placed by a catheter within a vein, artery, or other tubular shaped body organ for treating conditions such as, for example, occlusions, stenoses, aneurysms, dissection, or weakened, diseased, or abnormally dilated vessel or vessel wall, by expanding the vessel or by reinforcing the vessel wall. Stents can improve angioplasty results by preventing elastic recoil and remodeling of the vessel wall and treating dissections in blood vessel walls caused by balloon angioplasty of coronary arteries.

While conventional stent technology is relatively well developed, stent technologies related to treatment of the region of a vessel bifurcation are still being developed. One challenge related to treatment of a vessel bifurcation involves alignment of the stent relative to the vessel branch of the vessel bifurcation. Maintaining proper relative positioning of features of the catheter assembly used to deliver and deploy the stent at the vessel bifurcation can be an important aspect of achieving the desired stent alignment.

WO 95/11400 discloses a method and articles for holding fluid conduits at a desired lateral distance from each other for connection to spaced-apart connection points, for example vehicle fuel line connections or domestic appliance flexible hoses.

### SUMMARY

The invention is defined by the features of the independent claims. The examples, aspects or embodiments of the present description that do not fall within the scope of said claims are provided for illustrative purposes only and do not form part of the present invention.

The illustrated examples disclosed herein relate generally to catheter assemblies and related methods for maintaining relative positioning of various catheter shafts of a catheter assembly. One application that can benefit from the principles disclosed herein is treatment of vessel bifurcations. A vessel bifurcation can be defined as an area of a vessel in which at least one branch vessel diverts or branches away from a main vessel. An example catheter assembly configured for treatment of a vessel bifurcation carries a stent to the bifurcation treatment site. Some catheter assemblies include features that orient the stent relative to the branch vessel of the vessel bifurcation and maintain that orientation during treatment of the vessel bifurcation. An example catheter assembly includes a main catheter branch and a side catheter branch. Typically, a main balloon is positioned at a distal end portion of the main catheter shaft. The main balloon extends from a distal open end to a proximal open end of the stent. The side catheter branch extends through the proximal open end of the stent and out of a side opening of the stent. A distal end portion of the side catheter branch extends into a branch vessel at the vessel bifurcation and is used to help orient the side opening of the stent relative to the branch vessel. Maintaining a fixed radial and axial position of the side catheter branch relative to the main catheter branch along a length of the side catheter branch can help maintain proper alignment of the stent side opening relative to the branch vessel. The relative position of the side catheter branch to the main catheter branch can be maintained using, for example, various bonding and other securing techniques.

There is no requirement that an arrangement include all features characterized herein to obtain some advantage according to this disclosure.

### DESCRIPTION OF THE DRAWINGS

Figure 1 is a schematic side view of an example catheter assembly in accordance with principles of the present disclosure, wherein the main and side catheter branches are secured together proximal of the stent and the balloon members are deflated.
Figure 2 is a schematic side view of the catheter assembly shown in Figure 1, wherein the balloon members are inflated.
Figure 3 is a schematic side view of an example catheter shaft bond arrangement in accordance with the present disclosure.
Figure 4 is a schematic cross-sectional view of the bond arrangement shown in Figure 3 taken along cross-sectional indicators 4-4.
Figure 5 is a schematic cross-sectional view of the bond arrangement shown in Figure 3 after heat has been applied and a heat sleeve removed.
Figure 6 is a schematic side view of another example catheter shaft bond arrangement in accordance with the present disclosure.
Figure 7 is a schematic perspective view of the bonding extrusion shown in Figure 6.
Figure 8 is a schematic cross-sectional view of the bond arrangement shown in Figure 6 taken along cross-sectional indicators 8-8.
Figure 9 is a schematic cross-sectional view of the bond arrangement shown in Figure 6 after heat has been applied and a heat sleeve removed.
Figure 10 is a schematic side view of another example catheter shaft bond arrangement in accordance with the present disclosure.
Figure 11 is a schematic cross-sectional view of the bond arrangement shown in Figure 10 taken along cross-sectional indicators 10-10.
Figure 12 is a schematic cross-sectional view of the bond arrangement shown in Figure 10 after heat has been applied and the heat sleeve removed.
Figure 13 is a schematic side view of an example bond arrangement that includes a laser assembly.
Figure 13A and 13B are schematic views of example weld area shapes.

### DETAILED DESCRIPTION

This disclosure relates to catheter assemblies and related methods for maintaining relative positioning of various catheter shafts of a catheter assembly. The disclosed catheter assemblies and related methods include a main catheter shaft and a branch catheter shaft. A main balloon and a stent are typically positioned at a distal end portion of the main catheter shaft. The branch catheter shaft can be used to help orient the stent relative to a branch vessel at a vessel bifurcation. Maintaining a fixed axial and radial position of the branch catheter shaft relative to the main catheter branch can provide improved consistency in orienting the stent relative to the branch vessel prior to and during inflation of the balloon to expand the stent. The branch catheter shaft can be secured to the main catheter shaft using any of a variety of techniques such as, for example, heat bonding and adhesives.

The use of adhesives to secure the main and branch catheter shafts together can have certain disadvantages in performance and manufacturing. Adhesives are susceptible to the formation of voids that can cause failure of the bond joint. It can be difficult to inspect for voids and other problems with adhesive bonds during the manufacturing process. Making an adhesive joint can also be time consuming and require manufacturing of batches of product due to the time and space required for applying and curing the adhesive.

Securing catheter shafts together using heat bonding techniques can have advantages over adhesives. When securing together two polymer-based materials, such as those materials typically used for catheter shafts (see below), with a heat bond, cross-linking of the polymer chains of the two materials creates a physical bond. Heat bonds are typical stronger and more durable than a bond created using adhesives because of the cross-linking of the polymer chains in a heat bond verses bonding of the adhesive to each of the materials in an adhesive bond.

Further, a heat bond can be made relatively quickly using any one of several heat sources. Some example heat sources include a hot jaw, carbon dioxide lasers, and diode lasers. Different types of heat sources can dictate the additional features and steps required to create the heat bond. For example, using a hot jaw to create a heat bond can be accomplished by heat bonding a bond member to both catheter shafts. A heat sleeve is typically used to hold the bond member in place while heat is applied by the hot jaw.

Creating a heat bond using a diode laser can be simpler in that the diode laser merely heats one or the other of the catheter shafts at the bond location to create the heat bond without the use of an added bond member or heat sleeve. However, because a heat bond created by a diode laser does not use added material from a bond member, extra care must be taken not to weaken the catheter shafts or provide a bond that has low tensile strength. Further details related to a hot jaw and laser heat sources (e.g., a diode laser heat source) are provided in the description below.

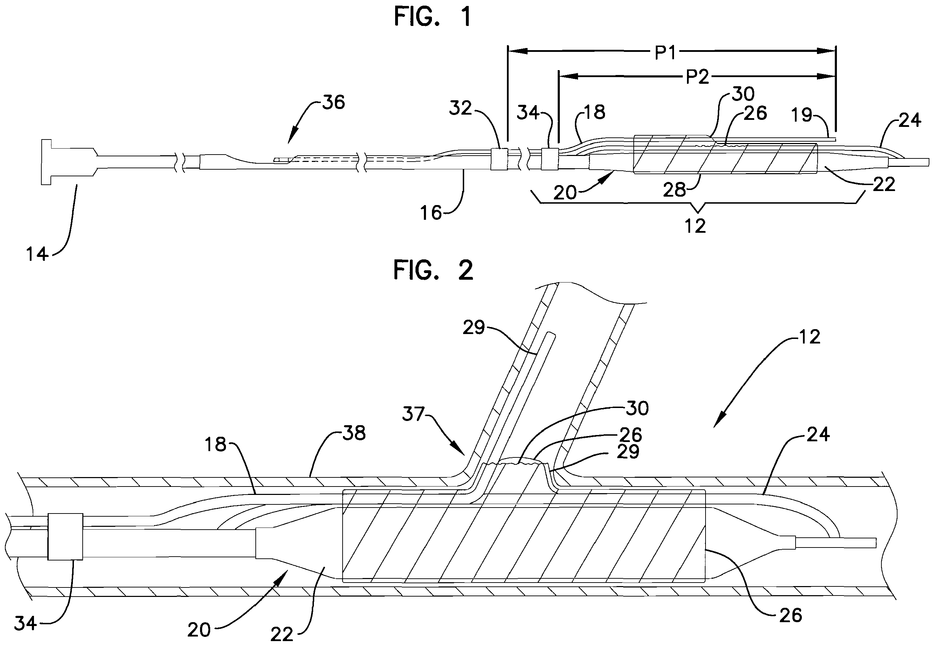

### I. The Catheter Assembly of Figures 1-2

Figures 1 and 2 illustrate a catheter assembly 10 for which aspects of the present disclosure can be applied. While alternatives are possible, the catheter assembly 10 includes a distal arrangement 12, a proximal end portion 14, and a catheter shaft 16 extending therebetween. The distal arrangement 12 includes a side catheter branch 18 and a main catheter branch 20. A proximal end portion of the side catheter branch 18 extends proximal of the distal arrangement 12 to a guidewire port arrangement 36. The side catheter branch 18 can extend within the main catheter shaft 16 along at least a portion of the length of the catheter shaft 16 proximal of the distal arrangement 12.

The main catheter branch 20 includes a main balloon 22, a side inflation member 24, a side balloon 26 positioned at a location along a length of the side inflation lumen 24, and a stent 28. The stent 28 includes a side opening 30 located at a position between distal and proximal open ends of the stent 28. A distal end portion of the side catheter branch 18 extends through the proximal open end of the stent 28 and out of the side opening 30. The main and side balloons 22, 26 maintain the deflated configuration shown in Figure 1 during advancement of distal arrangement 12 to a vessel treatment site, such as a vessel bifurcation 37 shown in Figure 2. At the vessel bifurcation 37, the side catheter branch 18 extends into a branch vessel 39 while the main catheter branch 20 remains within a main vessel 38. The side catheter branch 18 helps radially and axially align the stent side opening 30 relative to the branch vessel 39. When the stent 28 is properly positioned relative to the branch vessel 39, the main and side balloons 22, 26 are inflated to expand the stent 28 within the main vessel 38 and extend a plurality of extension members 29 of stent 28 into the branch vessel 39.

Maintaining a fixed axial and radial position of the side catheter branch 18 relative to the main catheter shaft 16 can help maintain the radial and axial position of the stent side opening 30 relative to the branch vessel 39 prior to and during inflation of the main and side balloons 22, 26 to expand the stent 28. At least one shaft bond arrangement is provided proximal of the main catheter branch 20 to help fix the axial and radial position of the side catheter branch 18 relative to the main catheter shaft 16.

Figure 1 illustrates first and second shaft bond arrangements 32, 34 spaced axially from each other at a location proximal of the main catheter assembly 12 and distal of the guidewire port arrangement 36. The first and second shaft bond arrangements 32, 34 are spaced distances P1, P2, respectively, proximally of a distal tip of the side catheter branch 18. In one example arrangement, the distance P1 is in the range of about 150 to 250 mm, and more preferably about 215 to about 225 mm. The distance P2 in one example arrangement can be in the range of about 50 to about 150 mm, and more preferably about 120 to about 130 mm. Alternative distances P1, P2 are possible. Any number of shaft bond arrangements can be used and spacing of the various bond arrangements can vary along a length of the side catheter branch 18 to maintain the desired axial and radial position of the side catheter branch 18 relative to the main catheter shaft 16.

The shaft bond arrangements 32, 34 and other shaft bond arrangements disclosed hereinafter are provided in order to achieve at least some of the following objectives. Preferably, the shaft bond arrangement provides a reliable connection between the side catheter branch 18 and main catheter shaft 16 that will not fail during use of the catheter assembly. The shaft bond arrangement preferably does not significantly increase the maximum outer profile of the catheter assembly as compared to the axially adjacent portions of the main and side catheter branches 16, 18 that do not include the shaft bond arrangement. Further, the shaft bond arrangement preferably does not significantly increase or alter the stiffness of the main and side catheter branches 16, 18 upon application of exterior applied forces such as bending and torsional forces. As will be discussed further below, the shaft bond arrangement can be provided in longitudinally adjacent arranged segments that help minimize increases in stiffness resulting from the shaft bond arrangement. The shaft bond arrangements preferably also do not alter the dimensions of the interior lumens defined by each of the main and side catheter branches 16, 18. These and other advantages and objectives are met and described in further detail in the description hereinafter.

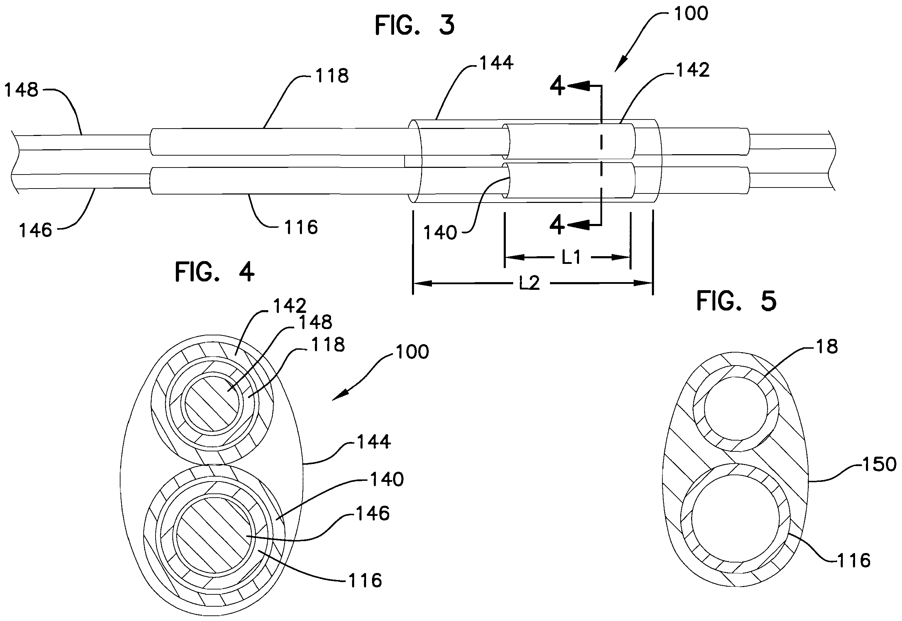

### II. The Bond Arrangement of Figures 3-5

An example shaft bond arrangement 100 is shown and described with reference to Figures 3-5. The shaft bond arrangement 100 can also be referred to as build-up shaft bond arrangement 100. The shaft bond arrangement 100 includes a main catheter shaft 116, a branch catheter shaft 118, first and second bonding segments 140, 142, a heat sleeve 144, and main and branch mandrels 146, 148. Each of the first and second bonding segments 140, 142 has a length L1, although in other arrangements each bonding segment can have a different length. The length L1 is typically in the range of about 2 mm to about 20 mm, and more preferably in the range of about 4 mm to about 10 mm. The first bonding segment 140 defines an internal lumen sized to receive the main catheter shaft 16. The second bonding segment 142 defines an internal lumen sized to receive the branch catheter shaft 118.

The heat sleeve 144 can be used to retain the first and second bonding segments 140, 142 in engagement with each other prior to and during application of heat via a heat source. The heat sleeve 144 has a length L2 that is typically greater than the length L1. The heat sleeve 144 typically includes a size and a material composition that provides radially inward shrinking of the heat sleeve 144 when heated. Shrinking of the heat sleeve 144 applies a radially inward directed constricting force upon the first and second bonding segments 140, 142. The application of a constricting force upon the first and second bonding segments 140, 142 when a source of heat is applied to the bonding segments 140, 142 can help reduce the amount of time required to complete the heat bond and increase the integrity of the resulting heat bond. An example heat shrink material is RayChem brand material made by Tyco Electronics of Menlo Park, CA.

The use of bonding segments 140, 142 that completely encapsulate an outer circumference of the main and branch catheter shafts 116, 118 can have certain advantages. One such advantage is that the catheter shafts 16, 18 are protected by the bonding segments from being overheated to a point where the catheter shafts themselves begin to reach a melting point. If the catheter shafts 16, 18 were heated to a melting point, the structural integrity, shape, and other characteristics of the catheter shafts 16, 18 could be affected adversely. The shape and size of the bonding segments 140, 142 also provide that there is sufficient material in the space between the catheter shafts 16, 18 to create a reliable bond between the catheter shafts 16, 18 (e.g., see the final molded bond 150 shown in Figure 5). The molded bond 150 extends not only between the catheter shafts 16, 18, but also encapsulates an entire outer periphery of each of the catheter shafts 16, 18.

The heat sleeve 144 comprises a material that does not bond to the bonding segments 140, 142 or the catheter shafts 116, 118. Typically, the heat sleeve 144 shrinks in size (e.g., length and internal volume) upon application of heat on an exterior surface of the heat sleeve 144. Typically, the heat sleeve 144 is removed after the molded bond 150 is cured as shown in Figure 5. An example method of removing the heat sleeve is to skive off the heat sleeve from the molded bond 150 and the catheter shafts 116, 118.

The following is an example method of creating a shaft bond arrangement in accordance with the features described above with reference to Figures 3-5.
- Load the main and branch catheter shafts 116, 118 onto respective main and branch mandrels 146, 148. Preferably, the mandrels 146, 148 comprise a stainless steel material and have an outer dimension substantially the same as an inner dimension of the lumens defined by each of the respective main and branch catheter shafts 116, 118. The mandrels 146, 148 preferably include a coating (e.g., hydrophilic polymer, a fluoropolymer such as polytetrafluoroethylene (PTFE-better known as TEFLON®), or other suitable material) that promotes easy removal of the mandrels from the catheter shafts 116, 118.
- Prepare the first and second bonding segments 140, 142 having a length of about, for example, 4 mm to about 6 mm each. The bonding segments 140, 142 can include a cut that extends between opposing ends of the bonding segments 140, 142. Such a cut can permit the bonding segments to be fit onto the main and branch catheter shafts 116, 118 at any position along the length of the shafts 116, 118. When such a cut is not provided, the shafts 116, 118 are inserted through the internal lumen defined by each of the bonding segments 140, 142 and the bonding segments 140, 142 are slid into proper axial positions on the shafts 116, 118.
- Ensure that both of the bonding segments 140, 142 are positioned at the proper axial position along the length of the shafts 116, 118 and aligned with each other.
- Position a heat sleeve member having a length of about 10 mm over the bonding segments 140, 142. Preferably, an equal amount of the heat sleeve extends on opposing sides of the bonding segments.
- Place the entire assembly into a hot jaw bonder that is equipped with elliptical jaws that have been heated to about 250°C.
- Cycle the hot jaw machine, allowing the jaws to close over the heat sleeve and apply heat that bonds the bonding segments for about 5 to 6 seconds.
- After completion of the cycle, remove the assembly from the hot jaw bonder and remove the heat sleeve from the resulting molded bond.

While specific materials, shapes and sizes of features, and amounts of time have been given in this example, variations on these aspects of the method are possible to provide the same or similar results of providing the desired heat bond.

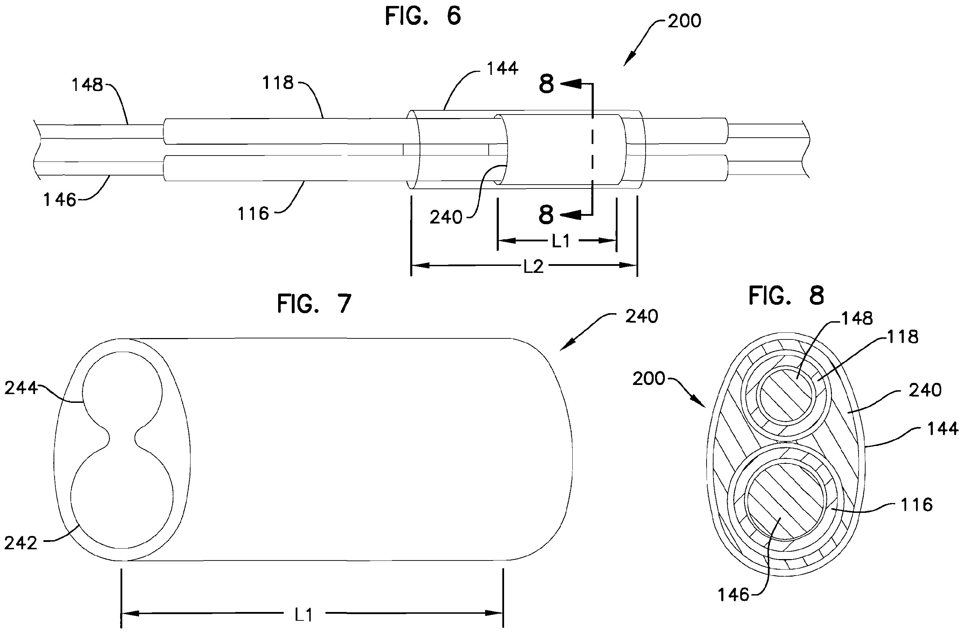

### III. The Bond Arrangement of Figures 6-9

Another example bond arrangement 200 is now described with reference to Figures 6-9. The bond arrangement 200 can also be referred to as a shaft fit extrusion bond arrangement in that the bond arrangement utilizes a single bonding extrusion member that defines separate bores sized to receive the catheter shafts 116, 118. The bond arrangement 200 includes a main catheter shaft 116, a branch catheter shaft 118, a bonding extrusion 240, a heat sleeve 144, a main mandrel 146, and a branch mandrel 148.

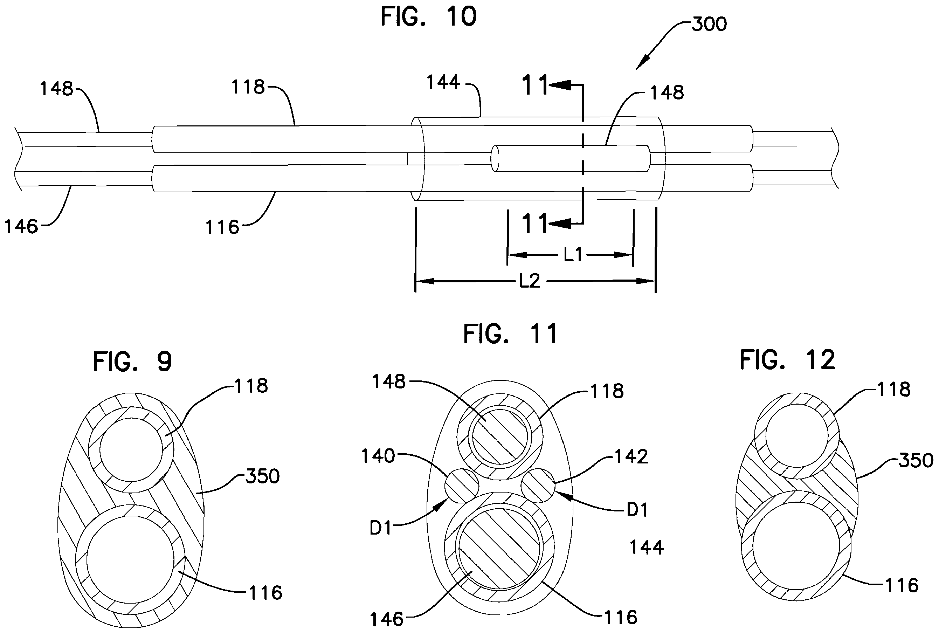

The bonding extrusion 240 defines a main shaft bore 242 sized to receive the main catheter shaft 116, and a branch shaft bore 244 sized to receive the branch catheter shaft 118. The bonding extrusion 240 has an outer circumference and cross-sectional shape of substantially the same size as the molded bond 250 (see Figure 9) that results from the application of the heat source to create the heat bond between the main and branch catheter shafts 116, 118. The bonding extrusion 240 has a length L1 that is typically less than a length L2 of the heat sleeve 144. In some examples, the bonding extrusion length L1 is in a range of about 2 to about 20 mm, and more preferably about 4 to about 10 mm. A length L1 that is too short may not be able to provide the desired amount of bonding and tensile strength to withstand failure during use of the main and branch catheter shafts 116, 118. A length L1 that is too long can impose an undesired amount of stiffness upon the main and branch catheter shafts 116, 118. In alternative arrangements, multiple segments of bonding extrusion can be positioned axially spaced apart along the main and branch catheter shafts to provide the desired amount of bonding and tensile strengths while still imposing limited stiffness.

As shown in Figure 8, the bond arrangement 200 includes the main and branch mandrels 146, 148 positioned within the main and branch catheter shafts 116, 118, respectively. The main and branch catheter shafts 116, 118 are positioned within the main and branch shaft bores 242, 244 of the bonding extrusion 240. The heat sleeve 144 is positioned around the bonding extrusion 240. The heat sleeve 144 is configured to provide a constricting force on the bonding extrusion 240 upon application of heat to the heat sleeve 144. When using some types of heat sources, the use of heat sleeve 144 may not be necessary.

Use of the bonding extrusion 240 can have certain advantages as compared to, for example, the separate first and second bonding segments 140,142 of the bond arrangement 100 described above. The bonding extrusion 240 can be properly positioned along both of the main and branch catheter shafts 116, 118 simultaneously. Any adjustments to the axial position of the bonding extrusion relative to the catheter shafts 116, 118 can be made easily for both of the catheter shafts 116, 118. Further, the bonding extrusion 240 is pre-shaped with a cross-sectional shape that closely matches the desired cross-sectional shape of the molded bond 250 after the bonding occurs. The bonding extrusion 240 has a cross-sectional shape that places a minimum amount of bonding material between and surrounding each of the main and branch catheter shafts 116, 118 such that the resulting molded bond 250 provides the desired bonding strength and structural integrity to resist failure, but does not unnecessarily increase the thickness of the bond arrangement 200.

The bonding extrusion 240 can be formed using different techniques such as, for example, extruding, injection molding, and casting. The bonding extrusion 240 can comprise any of a variety of polymeric or other materials that can bond with the catheter shafts 116, 118 upon application of heat.

An example method of using the shaft fit extrusion bond arrangement 200 in a process of constructing a catheter assembly is described:
- Load the main and branch catheter shafts onto the main and branch mandrels 146, 148, respectively. The mandrels 146, 148 can include a coating (e.g., Teflon or other type of material) that promotes easy removal of the mandrels after the heating process. The coated mandrels 146, 148 preferably have an outer dimension that is substantially the same as the internal dimension of each of the catheter shafts 116, 118, respectively.
- Prepare a shorter length of bonding extrusion 240 (e.g., about 4 mm to about 6 mm) and slide the bonding extrusion over the main and branch catheter shafts 116, 118 to a desired bonding location.
- Ensure that the bonding extrusion 240 is properly placed along the length of the catheter shafts 116, 118. Then slide a heat sleeve 144 over the bonding extrusion 240 such that equal amounts of the sleeve overhang on opposing ends of the bonding extrusion 240.
- Place the entire assembly into a hot jaw bonder that is equipped with elliptical jaws and that has been heated to, for example, about 250°C.
- Cycle the machine including closing the hot jaws over the heat shrink and apply heat for about 5-6 seconds or an amount to sufficiently re-flow the material of the bonding extrusion 240 to form a bond between the main and branch catheter shafts 116, 118.
- When the cycle is complete, remove the assembly from the hot jaw bonder and remove the heat shrink.

While a hot jaw bonder and other specific features and conditions are described in this example method, other heat sources, shapes and sizes and other conditions can be provided to result in a similar outcome using a bonding extrusion such as member 240 described above.

### IV. Bond Arrangement of Figures 10-12

Another example bond arrangement 300 is now described with reference to Figures 10-12. The bond arrangement 300 can also be referred to as a bead re-flow bond arrangement. The bond arrangement 300 includes main and branch catheter shafts 116, 118, first and second bonding beads 340, 342, a heat sleeve 144, and main and branch mandrels 146, 148. The bonding beads 340, 342 each have a length L1 and a diameter Dl. In other arrangements, the bonding beads 340, 342 can have different cross-sectional shapes besides the circular shape shown in Figure 11. The length L1 is typically about 2 mm to about 20 mm, and is less than the length L2 of the heat sleeve 144.

The use of bonding beads 340, 342 as the bonding material in the bond arrangement 300 can have advantages compared to, for example, the bonding segments 140, 142 and the bonding extrusion 240 described above. The bonding beads 340, 342 can provide a substantially smaller amount of bonding material as compared to the bonding segments 140, 142 and the bonding extrusion 240 described above. A reduction in the amount of total bonding material used in the bond arrangement can reduce the overall stiffness created as a result the heat bonding process (see molded bond 350 in Figure 12). Further, the bonding beads 340, 342 can be positioned at the specific location where the bonding between the main and branch catheter shafts 116, 118 is desired. Since the bonding beads 340, 342 are positioned at the juncture between the main and branch catheter shafts 116, 118, the resulting molded bond 350 does not increase the overall outer profile of the main and branch catheter shafts 116, 118, substantially. The bonding beads 340, 342 can be properly positioned relative to the catheter shafts 116, 118 without having to insert the catheter shafts 116, 118 into a bore, or having to make longitudinal cuts in the bonding beads 340, 342.

Another advantage of the bonding arrangement 300 relates to the heat sources used to create flow of the bonding beads 340, 342. When using a hot jaw, for example, the hot jaw can be configured to contact only the beads 340, 342 while having limited contact or no contact with a limited portion of each of the main and branch catheter shafts 116, 118. This focused application of heat reduces the possibility of creating overheating and other undesired changes to the main and branch catheter shafts 116,118.

An example method of bonding using the bead reflow bond arrangement 300 is now described:
- Load the main and branch catheter shafts 116, 118 onto the mandrels 146, 148, respectively. The mandrels 146, 148 can include a coating (e.g., Teflon or other material) that promotes easy removal of the mandrels from the catheter shafts after completion of the heat bonding process. Typically, the outer dimension of the coated mandrels is substantially the same size as the internal dimension of each of the catheter shafts.
- Prepare two lengths of bonding beads 340, 342 of a length of about, for example, about 4 mm to 6 mm. Position the beads 340, 342 on opposing sides of the main and branch catheter shafts 116, 118 and in axial alignment with each other.
- While ensuring that the beads 340, 342 are properly positioned along the catheter shafts 116, 118 and aligned with each other, the heat sleeve 144 is slid over the bonding beads 340, 342. Preferably, an equal amount of the heat sleeve 144 overhangs on opposing ends of each of the bonding beads 340, 342.
- Place the entire assembly in a hot jaw bonder that is equipped with elliptical jaws that have been heated to, for example, about 250°C.
- Cycle the hot jaw to close over the heat sleeve 144 and apply heat for about 5 to 6 seconds, or an amount of time sufficient to reflow the material of the bonding beads 340, 342 to create the bond 350 with the main and branch catheter shafts 116, 118.
- Remove the assembly 300 from the hot jaw bonder when the cycle is complete, and remove the heat sleeve 144.

While a specific heat source, and other features and variables have been specified in the above example, other applications can include different heat sources, features and characteristics of the components used to provide the same or similar type bead reflow bond arrangement.

### V. Example Heat Sources

Many different types of heat sources can be used to create the flow of material needed to create a heat generated bond between the main and branch catheter shafts for the bond arrangements described above. A hot jaw bonder has been described in the examples discussed above with reference to Figures 3-12. A hot jaw bonder can be configured with various contact surface shapes and sizes that provide a desired resultant cross-sectional shape of the molded bond and the application of heat to a specific location of the bond arrangement. The operation and advantages of hot jaw bonders are well known.

Another heat source that can be advantageous for use in the bond arrangements disclosed herein is a carbon dioxide laser. A carbon dioxide laser can have advantages as compared to the other example heat sources described herein.

A further heat source that can have particular advantages for use with the bond arrangements disclosed herein is a diode laser. Generally, in laser welding of thermoplastics, such as those polymer materials commonly used in catheter shafts, is sometimes referred to as laser transmission welding or IR welding, in which transparent and absorbing polymer parts are bonded together. The laser beam penetrates the transparent polymer and is converted to heat in the colored/absorbing polymer. Since both of the polymer parts are pressed together during the welding process, heat is conducted from the colored/absorbing polymer to the transparent polymer, allowing both materials to flow and create a heat generated bond. Internal joining pressure is also generated through local warming and thermal expansion. The internal and external joining pressures ensure strong penetration welding of both of the polymer parts.

Most thermoplastic and thermoplastic materials can be welded using diode laser radiation. For example, many if not all of the example materials listed below, even those materials reinforced with glass fibers, are suitable materials that provide a welding seam strength that is comparable to that of the base polymer material itself.

The use of diode lasers for a heat bond provide a number of advantages including a non-contact, flexible joining technique that results in minimal thermal stress on the polymer parts that are welded together. A diode laser provides a simple joining seam geometry without particulate development. A diode laser bond is a vibration-free process that provides optimal welding seam construction with high precision and a resulting high strength bond. The resultant seal generated between the various polymer parts is gas-tight and hermetic. Typically, there is no toolware involved nor any consumable material used (e.g., adhesives, fasteners, etc.) when using a diode laser to create the desired thermobond of catheter shafts.

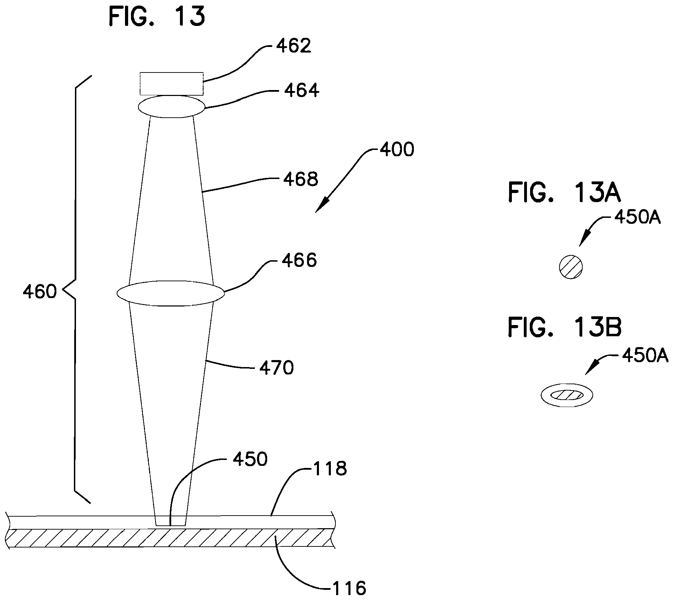

An example diode bonding arrangement and related processes is described now with reference to Figure 13. The bonding arrangement 400 includes first and second catheter members 116, 118 with a weld area 450 defined therebetween. A laser arrangement 460 includes a fiber optic cable 462, a beam enlarging lens 464, and a culminating or cylindrical lens 466. The beam expanding lens 464 generates an expanded beam that is directed to the lens 466. In the arrangement in which the lens 466 is a culminating lens, the resulting focus beam 470 has a generally cylindrical shape, such as the weld area 450A as shown in Figure 13A. The size of the beam at the weld area is approximately 0.7 mm in diameter in one example. The size of the beam can vary in other arrangements. Typically, either the catheter shafts 116,118 move back and forth relative to a fixed beam 470, or the entire laser assembly 460 is moved back and forth relative to the catheter shafts 116, 118.

In an example where the lens 466 is a cylindrical lens, the resulting focused beam 470 has an oval shape at the weld area 450. The beam in one example can have a maximum width dimension of 5.0 mm. In some arrangements, the maximum width dimension of the beam 470 of Figure 13B is approximately the same as the maximum width dimension of the resultant weld area 450B (see Figure 13B). Other beam shapes and sizes are possible as well as additional or fewer features provided in the diode laser arrangement.

An example method of bonding two catheter shafts using a diode laser is now described:
- A coherent quarto FAP-system fiber delivered diode laser assembly is set up. Pressure is applied to the catheter shafts to maintain contact between the catheter shafts.
- The diode laser is applied to the catheter shafts. Either the catheter shafts or the laser assembly is moved as needed to provide the desired weld area.
- The beam angle at which the beam engages the catheter shafts can vary for optical bonding. The beam geometry can vary as well for optimum bonding.
- During the bond process, the laser beam is oriented such that it transmits through the catheter shaft 118, which comprises a substantially transparent material, and contacts the colored catheter shaft 116. The colored catheter shaft absorbs the laser and is melted at least at its exterior surface. Melting of the catheter shaft 116 results in melting of the exterior surface of the abutting second catheter shaft 118 due in part to pressure being applied between the catheter shafts 116, 118. After the heat bond is completed, the laser energy is shut off and the materials are cooled.

### VII. Materials and Other Considerations

Materials used in the balloons, catheter shafts, and other components of the catheter assemblies disclosed herein can be made of any suitable material including, for example, thermoplastic polymers, polyethylene (high density, low density, intermediate density, linear low density), various co-polymers and blends of polyethylene, ionomers, polyesters, polycarbonates, polyamides, poly-vinyl chloride, acrylonitrile-butadiene-styrene copolymers, polyether-polyester copolymers, and polyetherpolyamide copolymers. One suitable material is Surlyn , a copolymer polyolefin material (DuPont de Nemours, Wilmington, Del.). Still further suitable materials include thermoplastic polymers and thermoset polymeric materials, poly(ethylene terephthalate) (commonly referred to as PET), thermoplastic polyamide, polyphenylene sulfides, polypropylene. Some other example materials include polyurethanes and block copolymers, such as polyamide-polyether block copolymers or amide-tetramethylene glycol copolymers. Additional examples include the PEBAX^{®} (a polyamide/polyether/polyester block copolymer) family of polymers, e.g., PEBAX^{®} 70D, 72D, 2533, 5533, 6333, 7033, or 7233 (available from Elf AtoChem, Philadelphia, Pa.). Other examples include nylons, such as aliphatic nylons, for example, Vestamid L2101 IF, Nylon 11 (Elf Atochem), Nylon 6 (Allied Signal), Nylon 6/10 (BASF), Nylon 6/12 (Ashley Polymers), or Nylon 12. Additional examples of nylons include aromatic nylons, such as Grivory (EMS) and Nylon MXD-6. Other nylons and/or combinations of nylons can also be used. Still further examples include polybutylene terephthalate (PBT), such as CELANEX^{®} (available from Ticona, Summit, N.J.), polyester/ether block copolymers such as ARNITEL (available from DSM, Erionspilla, Ind.), e.g., ARNITEL^{®} EM740, aromatic amides such as Trogamid (PA6-3-T, Degussa), and thermoplastic elastomers such as HYTREL^{®} (Dupont de Nemours, Wilmington, Del.). In some embodiments, the PEBAX^{®}, HYTREL^{®}, and ARNITEL^{®} materials have a Shore D hardness of about 45D to about 82D. The balloon materials can be used pure or as blends. For example, a blend can include a PBT and one or more PBT thermoplastic elastomers, such as RITEFLEX® (available from Ticona), ARNITEL^{®}, or HYTREL^{®}, or polyethylene terephthalate (PET) and a thermoplastic elastomer, such as a PBT thermoplastic elastomer. Additional examples of balloon material can be found in U.S. Pat. No. 6,146,356. It should be understood that the specific materials disclosed below for the individual embodiments does not limit the embodiment to those materials.

While the example catheter system 10 described above illustrates a balloon expandable stent having a predetermined branch aperture, other types of stents can be used with the catheter features described above. A variety of stents can be used with the systems and methods disclosed herein. Examples of such stents can be found in, for example, in U.S. Pat. Nos. 6,210,429, 6,325,826, and 7,220,275. In general, the aforementioned stents have a tubular shape with a continuous sidewall that extends between the proximal and distal ends. Proximal and distal stent apertures are defined at respective proximal and distal ends of the stent. A branch aperture is defined in the sidewall of the stent. The branch aperture provides access between an interior of the stent and an exterior of the stent. In some stents, the branch aperture includes expandable structure around a peripheral edge thereof that expands in a generally radial outward direction relative to a longitudinal axis of the stent. The expandable structure can be configured to extend into the branch lumen of the bifurcation upon expansion of the stent. The stent includes a plurality of strut structures that define the sidewall. The struts are expandable from a first, unexpanded state to a second, expanded state. Typically, the stent is configured to maintain the expanded state. The struts define a plurality of cell openings or cells along a length of the stent. The size and shape of the cells is typically different than the size and shape of the branch aperture. The stent is typically expanded once the stent is properly positioned in the main lumen of the bifurcation with the branch aperture aligned radially and axially with an opening into the branch lumen. The stent, including the expandable structure surrounding the branch aperture, can be expanded with a single expansion or with multiple expansions using, for example, one or more inflatable balloons.

### V. Conclusion

One aspect of the present disclosure relates to a catheter shaft bond arrangement that includes a first catheter shaft, a second catheter shaft, and a first bonding member. The first catheter shaft defines a first lumen. The second catheter shaft defines a second lumen. The first bonding member is configured to bond with the first and second catheter shafts to create a molded bond upon application of heat to the first bonding member. The molded bond provides a fixed axial and radial orientation of the first and second catheter shafts relative to each other.

Another aspect of the present disclosure relates to a catheter shaft bond member. The bond member includes an elongate shaft of material, a first bore, and a second bore. The elongate shaft includes a polymeric material. The first bore is defined in the elongate shaft. The first bore has a first internal dimension sized to receive a first catheter shaft. The second bore is defined in the elongate shaft and includes a second internal dimension that is sized to receive a second catheter shaft. The polymeric material of the elongate shaft is configured to bond with the first and second catheter shafts upon application of heat to the elongate shaft.

A further aspect of the present disclosure relates to a method of connecting first and second catheter shafts together in a fixed axial and radial orientation relative to each other. The method includes positioning the first and second catheter shafts adjacent to each other, positioning a first bonding member adjacent to the first and second catheter shafts, and applying heat to the first bonding member, wherein the applied heat creates a heat bond between the first bonding member and the first and second catheter shafts. The heat bond provides fixed axial and radial orientation of the first and second catheter shafts relative to each other.

A still further aspect of the present disclosure relates to a method of creating a bond between first and second catheter shafts. The method includes engaging an outer surface of the first catheter shaft with an outer surface of the second catheter shaft, and applying heat in the area of contact between the first and second catheter shafts, the applied heat creating a heat bond between the first and second catheter shafts.

The invention is set out in the following claims:

## Claims

1. A catheter shaft bond arrangement, comprising:
a first catheter shaft (16, 116) for deploying a stent at a patient's vessel bifurcation and defining a first lumen;
a second catheter shaft (18, 118) defining a second lumen; and
a first bonding member (32, 34, 100, 200, 300), the first bonding member (32, 34, 100, 200, 300) configured to re-flow and form a molded bond with the first and second catheter shafts (16, 18, 116, 118) upon application of heat to the first bonding member(32, 34, 100, 200, 300), the molded bond providing a fixed axial and radial orientation of the first and second catheter shafts (16, 18, 116, 118) relative to each other.

2. The catheter shaft bond arrangement of claim 1, wherein the first bonding member includes a first bore configured to receive a portion of the first catheter shaft, and a second bore configured to receive a portion of the second catheter shaft.

3. The catheter shaft bond arrangement of claims 1 or 2, further comprising a second bonding member, the second bonding member configured to re-flow and bond with the first and second catheter shafts to create the molded bond upon application of heat to the second bonding member.

4. The catheter shaft bond arrangement of claim 3, wherein the first bonding member extends around an outer peripheral surface of the first catheter shaft and the second bonding member extends around an outer peripheral surface of the second catheter shaft.

5. The catheter shaft bond arrangement of claim 3, wherein the first bonding member is positioned adjacent the first and second catheter shafts along a first side of the catheter shaft bond arrangement, and the second bonding member is positioned adjacent the first and second catheter shafts along a second side of the catheter shaft bond arrangement.

6. A method of connecting first and second catheter shafts (16, 18, 116, 118) together in a fixed axial and radial orientation relative to each other, the first catheter shaft being suitable for deploying a stent at a patient's vessel bifurcation,
the method comprising:
positioning the first and second catheter shafts (16, 18, 116, 118) adjacent to each other;
positioning a first bonding member (32, 34, 100, 200, 300) adjacent to the first and second catheter shafts (16, 18, 116, 118);
applying heat to the first bonding member (32, 34, 100, 200, 300) to re-flow the first bonding member and create a molded bond between the first bonding member (32, 34, 100, 200, 300) and the first and second catheter shafts (16, 18, 116, 118), the heat bond providing fixed axial and radial orientation of the first and second catheter shafts relative to each other.

7. The method of claim 6, further comprising positioning a second bonding member adjacent to the first and second catheter shafts along a side of the first and second catheter shafts opposite a position of the first bonding member.

8. The method of claims 6-7 further comprising applying heat to the second bonding member, wherein the applied heat creates the heat bond between the first bonding member and the first and second catheter shafts.

9. The method of claims 6-8 , wherein applying heat includes positioning the first bonding member and the first and second catheter shafts in a hot jaw and cycling the hot jaw to apply heat to the first bonding member.

10. The method of any of claims 6-9, wherein positioning the first bonding member includes sliding at least one of the first and second catheter shafts through a bore defined in the first bonding member.

11. The method of any of claims 6-10, wherein the first bonding member defines first and second bores, and positioning the first bonding member includes inserting the first catheter shaft into the first bore and inserting the second catheter shaft into the second bore.

12. The method of any of claims 6-11, wherein applying heat includes directing a laser beam into engagement with the first bonding member.

## Patentansprüche

1. Katheterschaftverbindungsanordnung, die aufweist:
einen ersten Katheterschaft (16, 116) zum Entfalten eines Stents an einer Gefäßverzweigung eines Patienten und der ein erstes Lumen definiert;
einen zweiten Katheterschaft (18, 118), der ein zweites Lumen definiert; und
ein erstes Verbindungselement (32, 34, 100, 200, 300), wobei das erste Verbindungselement (32, 34, 100, 200, 300) konfiguriert ist, bei der Anwendung von Wärme auf das erste Verbindungselement (32, 34, 100, 200, 300) wiederaufzuschmelzen und eine geformte Verbindung mit dem ersten und zweiten Katheterschaft (16, 18, 116, 118) zu bilden, wobei die geformte Verbindung eine feste axiale und radiale Ausrichtung des ersten und zweiten Katheterschafts (16, 18, 116, 118) relativ zueinander bereitstellt.

2. Katheterschaftverbindungsanordnung nach Anspruch 1, wobei das erste Verbindungselement ein erstes Loch, das konfiguriert ist, einen Abschnitt des ersten Katheterschafts aufzunehmen, und ein zweites Loch aufweist, das konfiguriert ist, einen Abschnitt des zweiten Katheterschafts aufzunehmen.

3. Katheterschaftverbindungsanordnung nach Anspruch 1 oder 2, die ferner ein zweites Verbindungselement aufweist, wobei das zweite Verbindungselement konfiguriert ist wiederaufzuschmelzen und sich mit dem ersten und zweiten Katheterschaft zu verbinden, um bei der Anwendung von Wärme auf das zweite Verbindungselement die geformte Verbindung zu erzeugen.

4. Katheterschaftverbindungsanordnung nach Anspruch 3, wobei sich das erste Verbindungselement um eine Außenumfangsfläche des ersten Katheterschafts erstreckt und sich das zweite Verbindungselement um eine Außenumfangsfläche des zweiten Katheterschafts erstreckt.

5. Katheterschaftverbindungsanordnung nach Anspruch 3, wobei das erste Verbindungselement längs einer ersten Seite der Katheterschaftverbindungsanordnung benachbart zum ersten und zweiten Katheterschaft angeordnet ist, und das zweite Verbindungselement längs einer zweiten Seite der Katheterschaftverbindungsanordnung benachbart zum ersten und zweiten Katheterschaft angeordnet ist.

6. Verfahren zum Verbinden eines ersten und zweiten Katheterschafts (16, 18, 116, 118) miteinander in einer festen axialen und radialen Ausrichtung relativ zueinander, wobei der erste Katheterschaft zum Entfalten eines Stents an einer Gefäßverzweigung eines Patienten geeignet ist, wobei das Verfahren aufweist:
Anordnen des ersten und zweiten Katheterschafts (16, 18, 116, 118) benachbart zueinander;
Anordnen eines ersten Verbindungselements (32, 34, 100, 200, 300) benachbart zum ersten und zweiten Katheterschaft (16, 18, 116, 118);
Anwenden von Wärme auf das erste Verbindungselement (32, 34, 100, 200, 300), um das erste Verbindungselement wiederaufzuschmelzen und eine geformte Verbindung zwischen dem ersten Verbindungselement (32, 34, 100, 200, 300) und dem ersten und zweiten Katheterschaft (16, 18, 116, 118) zu erzeugen, wobei die Wärmeverbindung eine feste axiale und radiale Ausrichtung des ersten und zweiten Katheterschafts relativ zueinander bereitstellt.

7. Verfahren nach Anspruch 6, das ferner das Anordnen eines zweiten Verbindungselements benachbart zum ersten und zweiten Katheterschaft längs einer Seite des ersten und zweiten Katheterschafts gegenüber einer Position des ersten Verbindungselements aufweist.

8. Verfahren nach Anspruch 6 bis 7, das ferner das Anwenden von Wärme auf das zweite Verbindungselement aufweist, wobei die angewendete Wärme eine Wärmeverbindung zwischen dem ersten Verbindungselement und dem ersten und zweiten Katheterschaft erzeugt.

9. Verfahren nach Anspruch 6 bis 8, wobei das Anwenden von Wärme das Anordnen des ersten Verbindungselements und des ersten und zweiten Katheterschafts in einer heißen Klemmbacke und das periodische Durchlaufen der heißen Klemmbacke aufweist, um Wärme auf das erste Verbindungselement anzuwenden.

10. Verfahren nach einem der Ansprüche 6 bis 9, wobei das Anordnen des ersten Verbindungselements das Schieben von mindestens einem des ersten und zweiten Katheterschafts durch ein Loch aufweist, das im ersten Verbindungselement definiert ist.

11. Verfahren nach einem der Ansprüche 6 bis 10, wobei das erste Verbindungselement ein erstes und zweites Loch definiert, und das Anordnen des ersten Verbindungselements das Einsetzen des ersten Katheterschafts in das erste Loch und das Einsetzen des zweiten Katheterschafts in das zweite Loch aufweist.

12. Verfahren nach einem der Ansprüche 6 bis 11, wobei das Anwenden von Wärme das Richten eines Laserstrahls in einen Eingriff mit dem ersten Verbindungselement aufweist.

## Revendications

1. Agencement de liaison de tiges de cathéter, comprenant :
une première tige de cathéter (16, 116) permettant de déployer un stent dans une bifurcation d'un vaisseau d'un patient et définissant une première lumière ;
une deuxième tige de cathéter (18, 118) définissant une deuxième lumière ; et
un premier élément de liaison (32, 34, 100, 200, 300), ledit premier élément de liaison (32, 34, 100, 200, 300) étant prévu pour fluer et former une liaison moulée avec la première et la deuxième tiges de cathéter (16, 18, 116, 118) par application de chaleur sur le premier élément de liaison (32, 34, 100, 200, 300), la liaison moulée permettant d'obtenir une orientation axiale et radiale fixe de la première et de la deuxième tiges de cathéter (16, 18, 116, 118) l'une par rapport à l'autre.

2. Agencement de liaison de tiges de cathéter selon la revendication 1, où le premier élément de liaison comprend un première trou prévu pour recevoir une partie de la première tige de cathéter, et un deuxième trou prévu pour recevoir une partie de la deuxième tige de cathéter.

3. Agencement de liaison de tiges de cathéter selon la revendication 1 ou la revendication 2, comprenant en outre un deuxième élément de liaison, ledit deuxième élément de liaison étant prévu pour fluer et former une liaison avec la première et la deuxième tiges de cathéter afin de créer la liaison moulée par application de chaleur sur le deuxième élément de liaison.

4. Agencement de liaison de tiges de cathéter selon la revendication 3, où le premier élément de liaison s'étend autour d'une surface périphérique extérieure de la première tige de cathéter et le deuxième élément de liaison s'étend autour d'une surface périphérique extérieure de la deuxième tige de cathéter.

5. Agencement de liaison de tiges de cathéter selon la revendication 3, où le premier élément de liaison est adjacent à la première et à la deuxième tiges de cathéter sur un premier côté de l'agencement de liaison de tiges de cathéter, et le deuxième élément de liaison est adjacent à la première et à la deuxième tiges de cathéter sur un deuxième côté de l'agencement de liaison de tiges de cathéter.

6. Procédé de connexion d'une première et d'une deuxième tiges de cathéter (16, 18, 116, 118) suivant une orientation axiale et radiale fixe l'une par rapport à l'autre, la première tige de cathéter étant adaptée pour déployer un stent dans une bifurcation d'un vaisseau d'un patient, ledit procédé comprenant :
le positionnement de la première et de la deuxième tiges de cathéter (16, 18, 116, 118) de manière adjacente l'une à l'autre ;
le positionnement d'un premier élément de liaison (32, 34, 100, 200, 300) de manière adjacente à la première et à la deuxième tiges de cathéter (16, 18, 116, 118) ;
l'application de chaleur sur le premier élément de liaison (32, 34, 100, 200, 300) pour fluer le premier élément de liaison et créer une liaison moulée entre le premier élément de liaison (32, 34, 100, 200, 300) et la première et la deuxième tiges de cathéter (16, 18, 116, 118), la liaison à chaud permettant d'obtenir une orientation axiale et radiale fixe de la première et de la deuxième tiges de cathéter l'une par rapport à l'autre.

7. Procédé selon la revendication 6, comprenant en outre
le positionnement d'un deuxième élément de liaison de manière adjacente à la première et à la deuxième tiges de cathéter sur un côté de la première et de la deuxième tiges de cathéter, opposé à une position du premier élément de liaison.

8. Procédé selon les revendications 6 et 7, comprenant en outre l'application de chaleur sur le deuxième élément de liaison, la chaleur application créant la liaison à chaud entre le premier élément de liaison et la première et la deuxième tiges de cathéter.

9. Procédé selon les revendications 6 à 8, où l'application de chaleur comprend le positionnement du premier élément de liaison et de la première et de la deuxième tiges de cathéter dans une mâchoire chauffée et la commande cyclique de ladite mâchoire chauffée pour appliquer la chaleur sur le premier élément de liaison.

10. Procédé selon l'une des revendications 6 à 9, où le positionnement du premier élément de liaison comprend le coulissement de la première et/ou de la deuxième tiges de cathéter dans un trou défini dans le premier élément de liaison.

11. Procédé selon l'une des revendications 6 à 10, où le premier élément de liaison définit un premier et un deuxième trous, et où le positionnement du premier élément de liaison comprend l'insertion de la première tige de cathéter dans le premier trou et l'insertion de la deuxième tige de cathéter dans le deuxième trou.

12. Procédé selon l'une des revendications 6 à 11, où l'application de chaleur comprend le guidage d'un faisceau laser pour contacter le premier élément de liaison.
